# EUROPEAN PATENT APPLICATION

(11) **EP 2 319 443 A1**
(43) Date of publication of application: **11.05.2011**
(21) Application number: 10190322.7
(22) Date of filing: 08.11.2010
(51) Int. Cl.: A61B 18/14, A61B 17/00

(54) **Battery powered electrosurgery**

(30) Priority: 06.11.2009 US 613876
(71) Applicant: Tyco Healthcare Group, LP, Boulder, CO 80301 (US)
(72) Inventor: Keller, Craig A., Boulder, CO 80304 (US)
(74) Representative: HOFFMANN EITLE

(57) **Abstract**

An electrosurgical device is provided where the device includes a housing including a cavity defined therein for housing an electrosurgical energy source, a controller configured to control the output of the electrosurgical energy source, and a power supply configured to supply power to the electrosurgical energy source and the controller. The housing also includes an active port configured to be operatively coupled to an end effector wherein the end effector applies electrosurgical energy from the electrosurgical energy source to tissue. The device also includes a return port configured to be operatively coupled to a return pad to provide a return path for the electrosurgical energy applied to tissue.

## Description

### BACKGROUND

### 1. Technical Field

The present disclosure relates to apparatuses for providing energy to biological tissue and more particularly, to a portable electrosurgical device for providing energy to biological tissue.

### 2. Background of Related Art

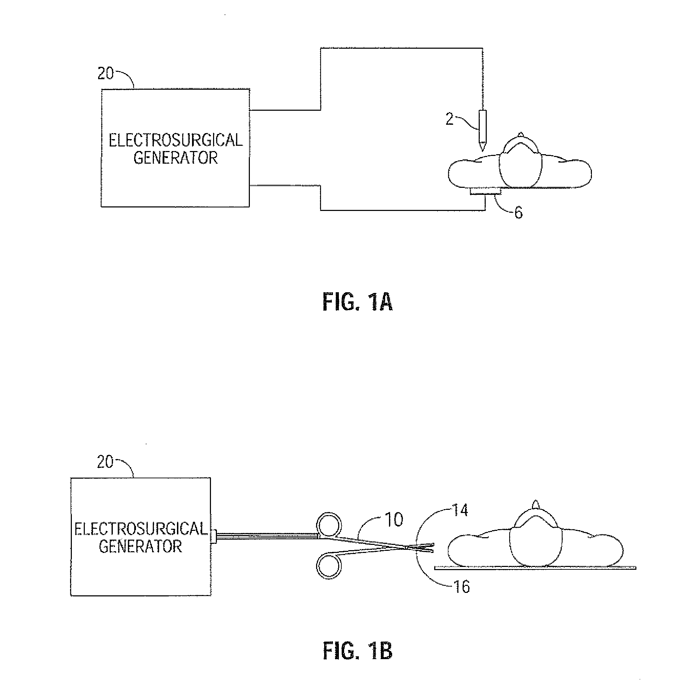

Energy-based tissue treatment is well known in the art. Various types of energy (e.g., electrical, ultrasonic, microwave, cryogenic, thermal, laser, etc.) are applied to tissue to achieve a desired result. Electrosurgery involves application of high radio frequency electrical current to a surgical site to cut, ablate, coagulate or seal tissue. In monopolar electrosurgery, as shown in Fig. 1A, a source or active electrode 2 delivers radio frequency energy from the electrosurgical generator 20 to the tissue and a return electrode 2 carries the current back to the generator. In monopolar electrosurgery, the source electrode is typically part of the surgical instrument held by the surgeon and applied to the tissue to be treated. A patient return electrode is placed remotely from the active electrode to carry the current back to the generator.

In bipolar electrosurgery, as shown in Fig. 1B, one of the electrodes of the hand-held instrument functions as the active electrode 14 and the other as the return electrode 16. The return electrode is placed in close proximity to the active electrode such that an electrical circuit is formed between the two electrodes (e.g., electrosurgical forceps 10). In this manner, the applied electrical current is limited to the body tissue positioned immediately adjacent to the electrodes. When the electrodes are sufficiently separated from one another, the electrical circuit is open and thus inadvertent contact with body tissue with either of the separated electrodes does not cause current to flow.

Electrosurgical instruments have become widely used by surgeons in recent years. By and large, most electrosurgical instruments are hand-held instruments, e.g., an electrosurgical pencil, which transfer radio-frequency (RF) electrical or electrosurgical energy to a tissue site. As used herein the term "electrosurgical pencil" is intended to include instruments which have a handpiece that is attached to an active electrode and which is used to cauterize, coagulate and/or cut tissue. Typically, the electrosurgical pencil may be operated by a handswitch or a foot switch. The active electrode is an electrically conducting element that is usually elongated and may be in the form of a thin flat blade with a pointed or rounded distal end. Alternatively, the active electrode may include an elongated narrow cylindrical needle that is solid or hollow with a flat, rounded, pointed or slanted distal end. Typically electrodes of this sort are known in the art as "blade", "loop" or "snare", "needle" or "ball" electrodes.

As mentioned above, the handpiece of the electrosurgical pencil is connected to a suitable electrosurgical energy source (i.e., generator) which produces the radio-frequency electrical energy necessary for the operation of the electrosurgical pencil. In general, when an operation is performed on a patient with an electrosurgical pencil, electrical energy from the electrosurgical generator is conducted through the active electrode to the tissue at the site of the operation and then through the patient to a return electrode. The return electrode is typically placed at a convenient place on the patient's body and is attached to the generator by a conductive material.

Some electrosurgical procedures utilize electrosurgical forceps that use both mechanical clamping action and electrical energy to affect hemostasis by heating tissue and blood vessels to coagulate, cauterize and/or seal tissue. As an alternative to open forceps for use with open surgical procedures, many modern surgeons use endoscopes and endoscopic instruments for remotely accessing organs through smaller, puncture-like incisions. As a direct result thereof, patients tend to benefit from less scarring and reduced healing time.

Endoscopic instruments are typically inserted into the patient through a cannula, or port, which has been made with a trocar. Typical sizes for cannulas range from three millimeters to twelve millimeters. Smaller cannulas are usually preferred, which, as can be appreciated, ultimately presents a design challenge to instrument manufacturers who must find ways to make endoscopic instruments that fit through the smaller cannulas. Such endoscopic instruments may use monopolar forceps, bipolar forceps or a combination monopolar/bipolar forceps.

Most electrosurgical procedures are performed in a hospital setting due to the need of a generator to supply energy to the electrosurgical instrument. Such generators tend to be large and generally expensive. Thus, electrosurgical procedures can not be performed in the field by first responders or military personnel nor can it be performed in a clinical setting where the purchase of a permanent generator is cost prohibitive.

### SUMMARY

In an embodiment of the present disclosure, an electrosurgical device is provided that includes a housing, in an embodiment a hand-held housing, including a cavity defined therein for housing an electrosurgical energy source, a controller configured to control the output of the electrosurgical energy source, and a power supply configured to supply power to the electrosurgical energy source and the controller. The housing may also include an active port configured to be operatively coupled to an end effector, wherein the end effector applies electrosurgical energy from the electrosurgical energy source to tissue, and a return port configured to be operatively coupled to a return pad to provide a return path for the electrosurgical energy applied to tissue.

The power supply for the electrosurgical device may be a battery which may be selectively replaceable or rechargeable. Further, the electrosurgical energy source outputs the electrosurgical energy in the form of a sine waveform, a square waveform, a pulse width modulated signal or a saw tooth waveform. The electrosurgical energy source is configured to output the electrosurgical energy, which output is powered by the power supply.

In another embodiment of the present disclosure, an electrosurgical pencil is provided having an elongated housing, in an embodiment a heldheld housing. The housing including a cavity defined therein for housing an electrosurgical energy source, a controller configured to control the output of the electrosurgical energy source and a power supply configured to supply power to the electrosurgical energy source and the controller. The housing may also include a return port configured to be operatively coupled to a return pad, an electrocautery electrode supported within the housing and extending distally from the housing, the electrocautery electrode being connected to the electrosurgical energy source and a plurality of activation switches supported on the housing, each activation switch being configured and adapted to selectively complete a control loop extending from the electrosurgical energy source upon actuation thereof. Preferably, the electrosurgical energy source is configured to output the electrosurgical energy in a waveform.

In the electrosurgical pencil, at least one activation switch is configured and adapted to control a waveform duty cycle to achieve a desired surgical intent. The pencil may also include three mode activation switches supported on the housing, wherein each mode activation switch delivers a characteristic signal to the source of electrosurgical energy which in turn transmits a corresponding waveform duty cycle to the electrosurgical pencil. A first activation switch delivers a first characteristic signal to the source of electrosurgical energy which in turn transmits a waveform duty cycle which produces a cutting effect, a second activation switch delivers a second characteristic signal to the source of electrosurgical energy which in turn transmits a waveform duty cycle which produces a blending effect, and a third activation switch delivers a third characteristic signal to the source of electrosurgical energy which in turn transmits a waveform duty cycle which produces a coagulating effect.

The power supply of the electrosurgical pencil may be a battery that is selectively replaceable and/or rechargeable.

In another embodiment of the present disclosure, an endoscopic forceps is provided having a housing, in an embodiment a handheld housing, having a shaft attached thereto. The housing including a cavity defined therein for housing an electrosurgical energy source, a controller configured to control the output of the electrosurgical energy source, a power supply configured to supply power to the electrosurgical energy source and the controller, and a return port configured to be operatively coupled to a return pad. The shaft includes a pair of jaw members disposed at a distal end thereof. The endoscopic forceps also includes a drive assembly disposed in the housing operable to move the jaw members relative to one another from a first position, wherein the jaw members are disposed in spaced relation relative to one another, to a second position, wherein the jaw members are closer to one another, for manipulating tissue. Each jaw member is adapted to connect to the electrosurgical energy source such that the jaw members are capable of conducting energy for treating tissue. A first switch is disposed on the housing and is activatable to selectively deliver energy of a first electrical potential to at least one jaw member for treating tissue in a monopolar fashion and a second switch is disposed on the housing and is activatable to selectively deliver energy of a first electrical potential to one jaw member and selectively deliver energy of a second electrical potential to the other jaw member for treating tissue in a bipolar fashion. Preferably, the electrosurgical energy source is configured to output the electrosurgical energy in waveform.

The power supply of the electrosurgical pencil may be a battery that is selectively replaceable or rechargeable.

### DRIEF DESCRIPTION OF THE DRAWINGS

The above and other aspects, features, and advantages of the present disclosure will become more apparent in light of the following detailed description when taken in conjunction with the accompanying drawings in which:

Figs. 1A-1B are schematic diagrams of electrosurgical systems;

Fig. 2 is a schematic block diagram of an electrosurgical system according to an embodiment of the present disclosure for use with various instrument types;

Fig. 3 is a schematic block diagram of an electrosurgical system according to another embodiment of the present disclosure for use with various instrument types;

Fig. 4 is a perspective view of an Electrosurgical pencil in accordance with an embodiment of the present disclosure;

Fig. 5 is a plan view of the electrosurgical pencil of Fig. 4;

Fig. 6 is a side, elevational view of the electrosurgical pencil of Fig. 4;

Fig. 7 is a partially broken away, side elevational view of the electrosurgical pencil of Fig. 4;

Fig. 8 is a front, elevational view of the electrosurgical pencil of Fig. 4;

Fig. 9 is a side, elevational view of an electrosurgical pencil according to an embodiment of the present disclosure;

Fig. 10 is a plan view of the electrosurgical pencil of Fig. 9;

Fig. 11 is a front, perspective view of a distal end portion of an electrosurgical pencil according to an embodiment of the present disclosure;

Fig. 12 is a front, perspective view of a distal end portion of an electrosurgical pencil according to an embodiment of the present disclosure;

Fig. 13 is an enlarged, perspective view of a portion of an electrosurgical pencil illustrating a set of switches disposed thereon;

Fig. 14 is an enlarged, perspective view of a portion of an electrosurgical pencil illustrating another set of switches disposed thereon; and

Fig. 15 is a perspective view of the switch of Fig. 14;

Fig. 16A is a top, perspective view of an endoscopic forceps shown in an open configuration and including a housing, a handle assembly, a shaft and an end effector assembly according to the present disclosure;

Fig. 16B is a top, perspective view of the endoscopic forceps of Fig. 16A showing the end effector assembly in a closed configuration according to the present disclosure;

Fig. 17 is a bottom, perspective view of the endoscopic forceps of Fig. 16A;

Fig. 18 is top, perspective view of the forceps of Fig. 16B showing rotation of the end effector assembly;

Fig. 19A is an enlarged, left perspective view of an end effector assembly;

Fig. 19B is an enlarged, left perspective view of an end effector assembly in a closed configuration;

Fig. 19C is an enlarged, side view of the end effector assembly;

Fig. 19D is an enlarged, end view of the end effector assembly;

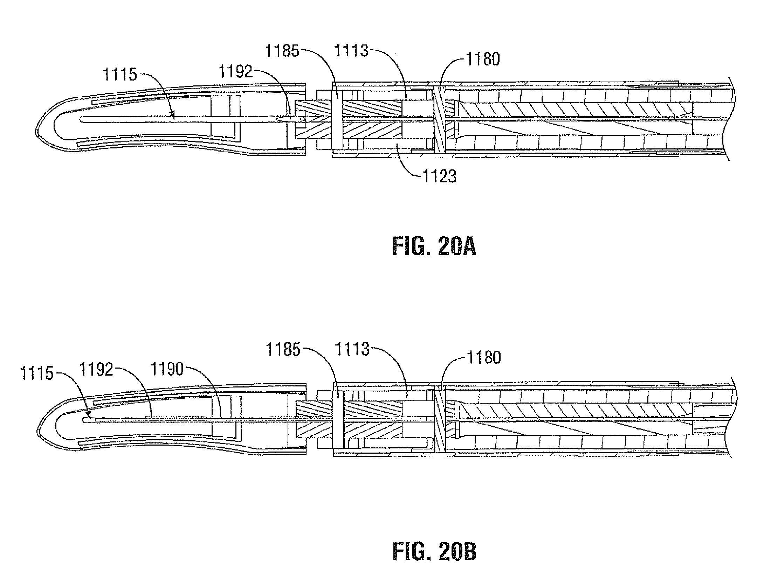

Fig. 20A is a greatly-enlarged, top cross sectional view of the end effector assembly showing a knife of the knife actuator in a proximal-most or unactuated position;

Fig. 20B is a greatly-enlarged, top cross sectional view of the end effector assembly of Fig. 31A showing the position of the knife after actuation;

Fig. 21A is a greatly-enlarged, side cross sectional view of the end effector assembly shown in an open configuration;

Fig. 21B is a greatly-enlarged, side cross sectional view of the end effector assembly shown in a closed configuration;

Fig. 21C is a greatly-enlarged, front perspective view of a bottom jaw member of the end effector assembly showing the knife of the knife actuator in a proximal-most or unactuated position;

Fig. 21D is a greatly-enlarged, front perspective view of the bottom jaw member of Fig. 21C showing the position of the knife after actuation;

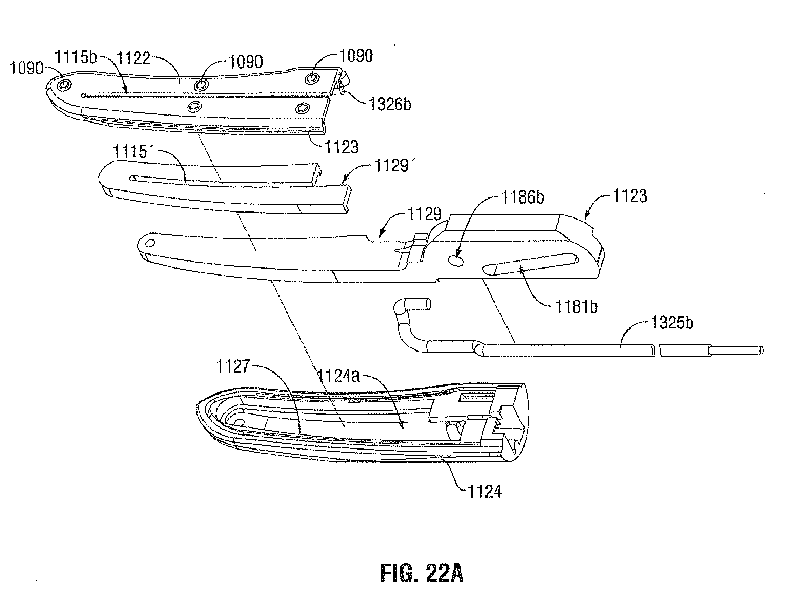

Fig. 22A is a greatly-enlarged, perspective view of the bottom jaw of the end effector assembly with parts separated; and

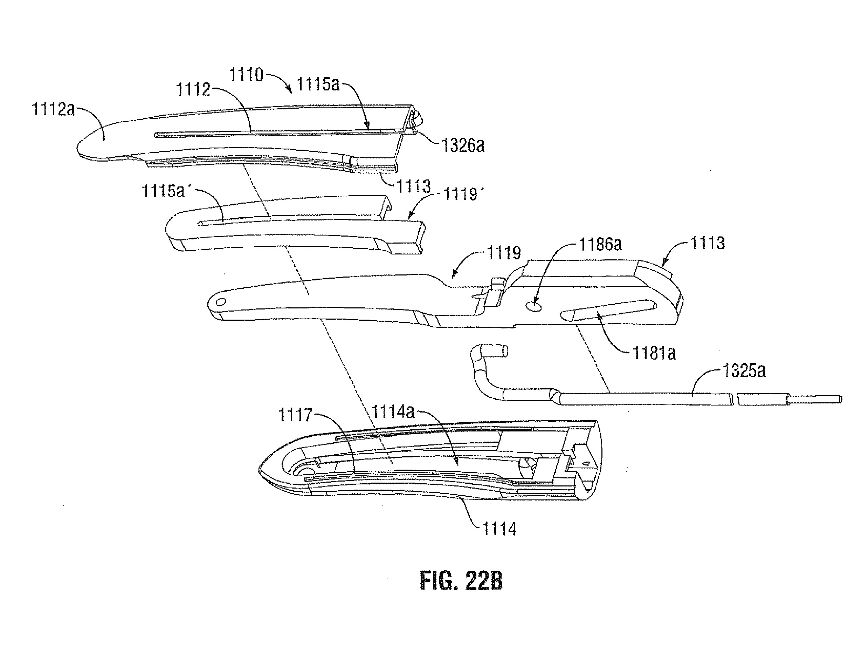

Fig. 22B is a greatly-enlarged, perspective view of the top jaw of the end effector assembly with parts separated.

### DETAILED DESCRIPTION

Particular embodiments of the present disclosure are described hereinbelow with reference to the accompanying drawings; however, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

Like reference numerals may refer to similar or identical elements throughout the description of the figures. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

Electromagnetic energy is generally classified by increasing energy or decreasing wavelength into radio waves, microwaves, infrared, visible light, ultraviolet, X-rays and gamma-rays. As used herein, the term "microwave" generally refers to electromagnetic waves in the frequency range of 300 megahertz (MHz) (3 x 10⁸ cycles/second) to 300 gigahertz (GHz) (3 x 10¹¹ cycles/second). As used herein, the term "RF" generally refers to electromagnetic waves having a lower frequency than microwaves.

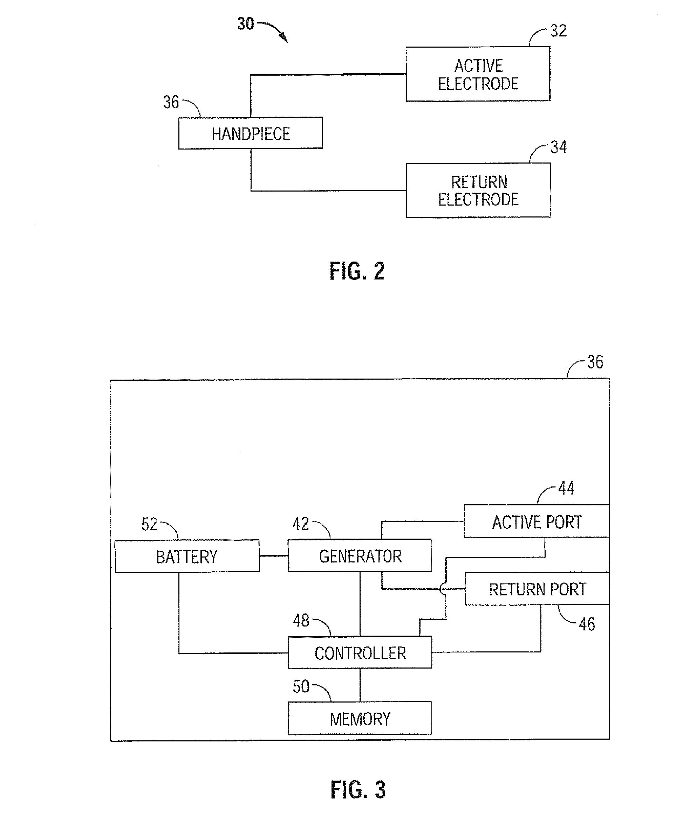

Fig. 2 shows a block diagram of an electrosurgical system 30 according to an embodiment of the present disclosure. As shown in Fig. 2, system 30 includes an active electrode 32, a return electrode 34 and a handpiece 36. Active electrode 32 is operatively coupled to handpiece 36 and may be an end effector such as monopolar forceps, bipolar forceps, a combination monopolar/bipolar forceps or a blade that may include a planar blade, a loop, a needle or the like. Return electrode 34 may be an RF return pad that is operatively coupled to handpiece 36. As such, electrosurgical system 30 is a portable device that is not limited for use in a hospital or clinical setting, but may also be used in the field by first responders and the military.

Return electrode 34 (or RF return pad 34) may have any suitable regular or irregular shape such as circular or polygonal. RF return pad 34 may be a conductive pad that may include a plurality of conductive elements arranged in a regular or irregular array. Each of the plurality of conductive elements may be equally-sized or differently-sized and may form a grid/array on the conductive pad. The plurality of conductive elements may also be arranged in a suitable spiral or radial orientation on the conductive pad. The use of the term "conductive pad" as described herein is not meant to be limiting and may indicate a variety of different pads including, but not limited to, conductive, inductive, or capacitive pads.

Fig. 3 shows a representative block diagram of the handpiece 36 according to an embodiment of the present disclosure. As shown in Fig. 3, handpiece 36 has a generator 42 that generates and outputs electrosurgical energy to the active port 44 that is coupled to an active electrode 32 or end effector (Fig. 2). The electrosurgical energy output from the generator 42 is sufficient to cut, cauterize, coagulate, seal or ablate tissue. The electrosurgical energy may be outputted as a sine waveform, square waveform, a pulse-width modulated (PWM) signal, a saw tooth waveform or any other waveform set by a manufacturer or a user that would accomplish the effects desired by the user of the electrosurgical device. The handpiece also has a return port 46 that receives energy from the return electrode 34.

The controller 48 may include a microcontroller operably connected to a memory 50, which may be volatile type memory (e.g., RAM) and/or non-volatile type memory (e.g., flash media, disk media, etc.). The microcontroller includes an output port that is operably connected to the generator 42 allowing the microcontroller to control the output of the microwave generator 42. Those skilled in the art will appreciate that the microcontroller may be substituted by any logic controller (e.g., control circuit) adapted to perform the calculations discussed herein. Memory 50 may be used to store a set of instructions, reference values, or other programming that may be used by the controller 48 to control the output of the generator 42. The controller may also include an input port configured to receive a signal from the active port 44 representative of the output energy and to receive a signal from the return port 46 representative of the return energy. Based on the signals from the active port 44 and return port 46, the controller may adjust the output of the generator 42.

The components in handpiece 36, such as generator 42 and controller 48, may be powered by a power supply or battery 52. Battery 52 may be a primary battery that transforms chemical energy to electrical energy or a secondary battery that can be recharged.

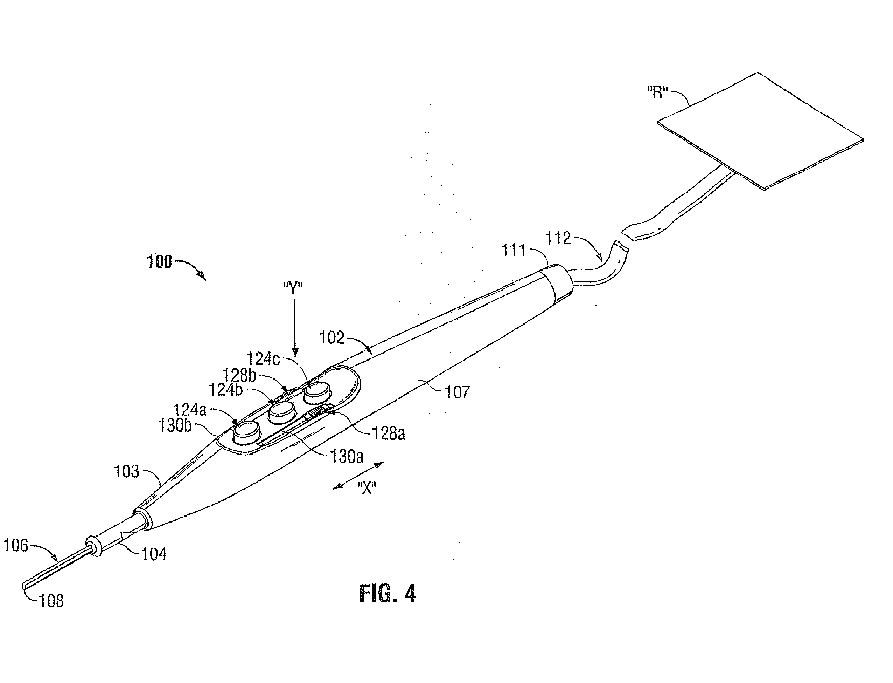

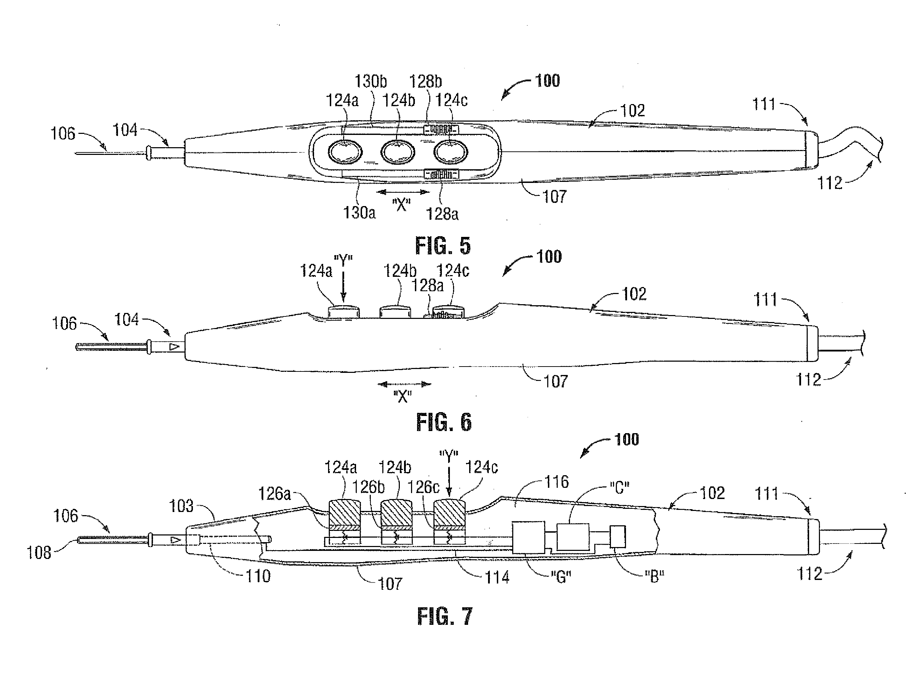

Turning now to Figs. 4-8, an electrosurgical pencil constructed in accordance with an embodiment of the present disclosure is shown generally as 100. Commonly owned U.S. Patent Application Serial No. 10/718,113 entitled "ELECTROSURGICAL PENCIL WITH 3-D CONTROLS" (now U.S. Patent No. 7,156,844), the contents of which are herein incorporated by reference in their entirety. Electrosurgical pencil 100 includes an elongated housing 102, which may be similar to handpiece 36 of Fig. 3, configured and adapted to support a blade receptacle 104 at a distal end 103 thereof which, in turn, receives a replaceable electrocautery end effector 106 in the form of a loop and/or blade therein. Electrocautery blade 106 is understood to include a planar blade, a loop, a needle and the like. A distal end portion 108 of blade 106 extends distally from receptacle 104 while a proximal end portion of blade 106 is retained within distal end 103 of housing 102. Electrocautery blade 106 may be fabricated from a conductive type material, such as, for example, stainless steel, or is coated with an electrically conductive material. The electrosurgical pencil also includes an electrosurgical energy source or generator "G", a controller "C" and a battery "B" (see Fig. 7).

As shown, electrosurgical pencil 100 is coupled to a return pad "R" via a cable 112. Cable 112 includes a transmission wire which electrically interconnects return pad "R" with return port 111 of electrosurgical pencil 100. Connecting return pad "R" directly to the electrosurgical pencil precludes the need for a separate generator and/or controller.

For the purposes herein, the terms "switch" or "switches" includes electrical actuators, mechanical actuators, electro-mechanical actuators (rotatable actuators, pivotable actuators, toggle-like actuators, buttons, etc.) or optical actuators.

Electrosurgical pencil 100 includes at least one activation switch, preferably three activation switches 124a-124c, each of which are supported on an outer surface 107 of housing 102. Each activation switch 124a-124c is operatively connected to a respective switch 126a-126c which, in turn, controls the transmission of RF electrical energy supplied from generator "G" to electrosurgical blade 106. More particularly, switches 126a-126e are electrically coupled to control loop 116 and are configured to close and/or complete control loop 116 to thereby permit RF energy to be transmitted to electrocautery blade 106 from electrosurgical generator "G".

Activation switches 124a-124c are configured and adapted to control the mode and/or "waveform duty cycle" to achieve a desired surgical intent in the same manner as activation switches 24a-24c of electrosurgical pencil 10 described above.

Electrosurgical pencil 100 further includes at least one intensity controller 128a and/or 128b, each of which are slidingly supported in guide channels 130a, 130b, respectively, which are formed in outer surface 107 of housing 102. Each intensity controller 128a and 128b is a slide-like potentiometer. It is contemplated that each intensity controller 128a and 128b and respective guide channel 130a and 130b may be provided with a series of cooperating discreet or detented positions defining a series of positions to allow easy selection of output intensity from a minimum amount to a maximum amount. The series of cooperating discreet or detented positions also provide the surgeon with a degree of tactile feedback. One of the series of positions for intensity controllers 128a, 128b may be an "off" position (i.e., no level of electrical or RF energy is being transmitted).

Intensity controllers 128a and 128b are configured and adapted to adjust one of the power parameters (e.g., voltage, power and/or current intensity) and/or the power verses impedance curve shape to affect the perceived output intensity.

For example, the greater intensity controllers 128a, 128b are displaced in a distal direction (i.e., in the direction of electrocautery blade 106) the greater the level of the power parameters transmitted to electrocautery blade 106. Conceivably, current intensities can range from about 60 mA to about 240 mA when using an electrosurgical blade and having a typical tissue impedance of about 2000 ohms. An intensity level of 60 mA provides very light and/or minimal cutting/dissecting/hemostatic effects. An intensity level of 240 mA provides very aggressive cutting/dissecting/hemostatic effects. Accordingly, the preferred range of current intensity is from about 100 mA to about 200 mA at 2K ohms.

The intensity settings may be preset and selected from a look-up table based on a choice of electrosurgical instruments/attachments, desired surgical effect, surgical specialty and/or surgeon preference. The selection may be made automatically or selected manually by the user. The intensity values may be predetermined or adjusted by the user.

In operation and depending on the particular electrosurgical function desired, the surgeon depresses one of activation switches 124a-124c, in the direction indicated by arrow "Y" (see Figs. 4 and 7) thereby closing a corresponding switch 126a-126c and closing and/or completing control loop 116. For example, the surgeon can depress activation switch 124a to perform a cutting or dissecting function, activation switch 124b to perform a dissecting/hemostatic function, or activation switch 124c to perform a hemostatic function. In turn, generator "G" transmits an appropriate waveform output to electrocautery blade 106 via transmission wire 114.

In order to vary the intensity of the power parameters of electrosurgical pencil 100, e.g., the current intensity, the surgeon displaces at least one of intensity controllers 128a, 128b in the direction indicated by double-headed arrow "X". As mentioned above, the intensity can be varied from approximately 60 mA for a light effect to approximately 240 mA for a more aggressive effect. For example, by positioning one of intensity controllers 128a, 128b closer to the proximal-most end (i.e., closer to cable 112) a light effect is produced and by positioning one of intensity controllers 128a, 128b closer to the distal-most end (i.e., closer to electrocautery blade 106) a more aggressive effect is produced. As described above, each intensity controller 128a, 128b can be configured and adapted to provide a degree of tactile feedback. Alternatively, audible feedback can be produced from each intensity controller 128a, 128b (e.g., a "click"), electrosurgical energy source "G" (e.g., a "tone") and/or an auxiliary sound-producing device such as a buzzer (not shown).

As shown in Fig. 7, electrosurgical pencil 100 may include a power source or battery "B", a controller "C" and an electrosurgical energy source or generator "G" within housing 102. Battery "B" supplies power to controller "C" and generator "G" and may be a primary battery or a secondary battery. Controller "C" receives inputs from the various switches, intensity controller, nubs, potentiometers or the like that may be disposed in housing 102 and outputs a signal to generator "G". Generator "G" provides electrosurgical energy based on the signal provided by controller "C".

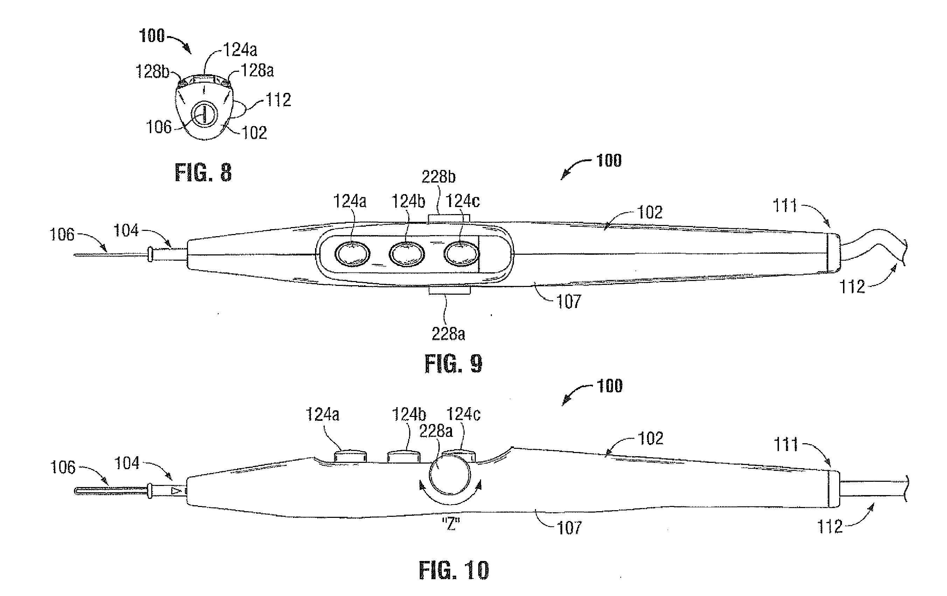

In an alternative embodiment, as seen in Figs. 9 and 10, sliding intensity controllers 128a, 128b have been replaced with intensity controllers 228a, 228b in the form of dial-like VDNs. Intensity controllers 228a, 228b function to vary the intensity of the power parameters via a rotation of dial controllers 228a, 228b in either a clockwise or counter-clockwise direction as indicated by double headed arrow "Z".

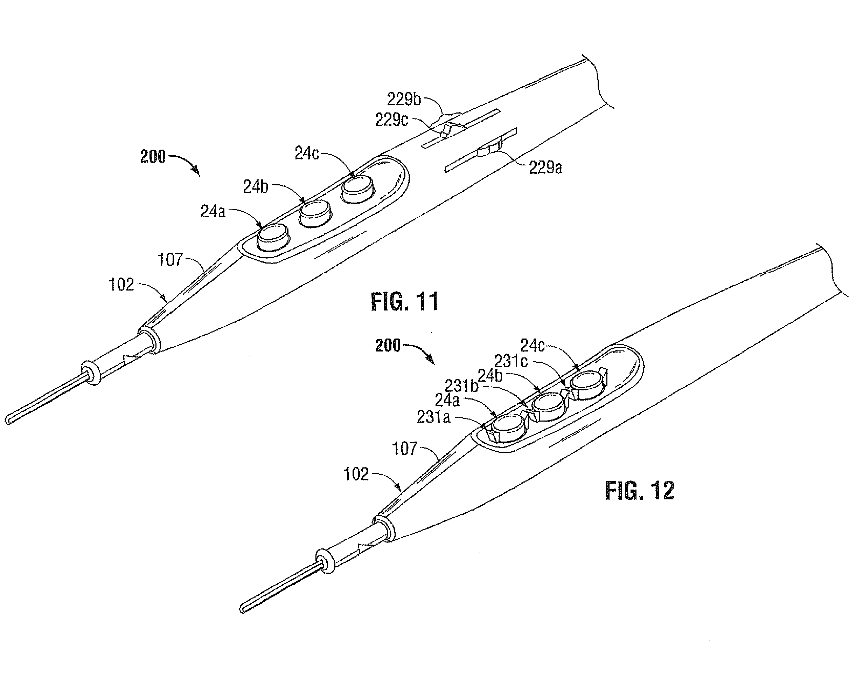

Fig. 11 depicts an alternative embodiment of an electrosurgical pencil shown generally as 200. Electrosurgical pencil 200 is similar to electrosurgical pencil 100 and will only be discussed in detail to the extent necessary to identify differences in construction and operation. As seen in Fig. 11, electrosurgical pencil 200 includes a plurality of nubs, e.g., three nubs, 229a-229c that are each operatively engaged with a slide potentiometer.

Accordingly, electrosurgical pencil 200 can be confgured such that each activation switch 24a-24c is a separate mode, such as, for example, activation switch 24a can be set such that electrosurgical pencil 200 performs "division" when depressed, activation switch 24b can be set such that electrosurgical pencil 200 performs "division with hemostasis" when depressed, and activation switch 24c can be set such that electrosurgical pencil 200 performs "hemostasis" when depressed. In addition, each nub 229a-229c is in operative engagement with a corresponding activation switch 24a-24c such that the power for each mode of operation of electrosurgical pencil 200 can be independently adjusted. As seen in Fig. 12, nubs 229a-229c of electrosurgical pencil 200 have been replaced with toggles 231a-231c operatively engaged with a respective activation switch 24a-24c. Each toggle 231a-231c can be operatively engaged with a rocker-type switch (not shown) or a rotational dial (not shown) in place of the slide-type potentiometer described above.

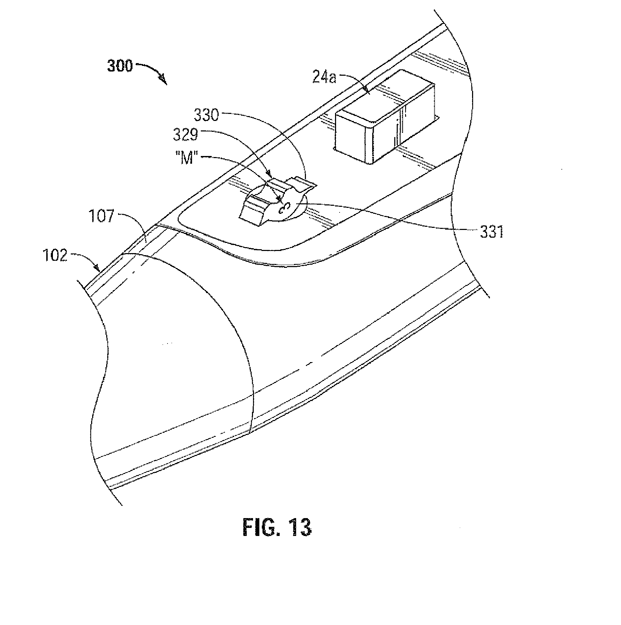

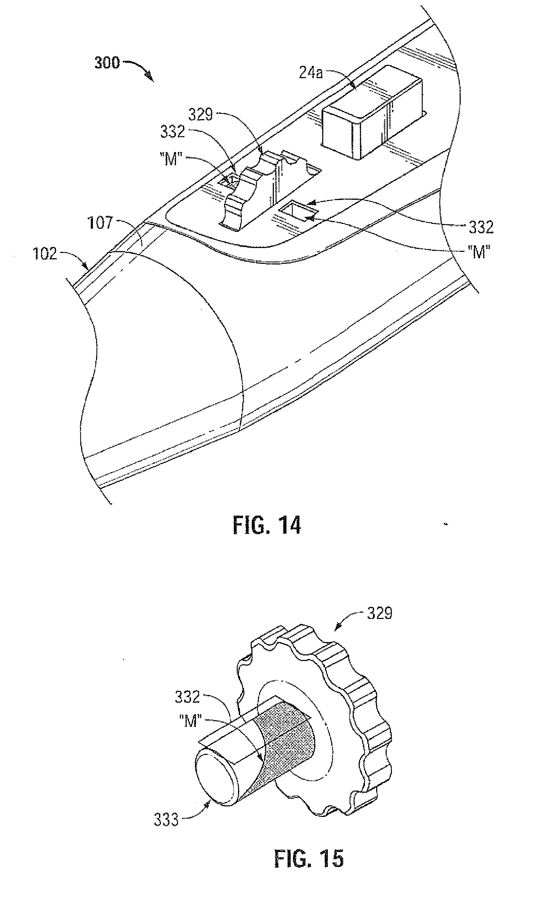

Turning now to Figs. 13-15, an electrosurgical pencil, in accordance with still another embodiment of the present disclosure, is generally designated as 300. Electrosurgical pencil 300 is similar to electrosurgical pencil 100 and will only be discussed in detail to the extent necessary to identify differences in construction and operation. As seen in Figs. 13 and 14, a dial 329 is rotatably supported in an aperture 330 formed in outer surface 107 of housing 102. A side surface 331 of dial 329 can be provided with indicia and/or markings "M" in the form of a scale and/or other form of gradient to indicate to the surgeon the degree of and/or level of power at which electrosurgical pencil 300 is set. As seen in Figs. 14 and 15, windows 332 can be formed on either side of dial 329 in outer surface 107 of housing 102. As seen in Fig. 15, windows 332 provide the surgeon with visibility to indicia "M" provided on stub 333 extending from the central axis of dial 329.

Embodiments of the present disclosure may also be incorporated into endoscopic instruments such as the instruments disclosed in commonly owned U.S. Patent Application Serial No. 11/540,335 entitled "IN-LINE VESSEL SEALER AND DIVIDER", the contents of which are herein incorporated by reference in their entirety.

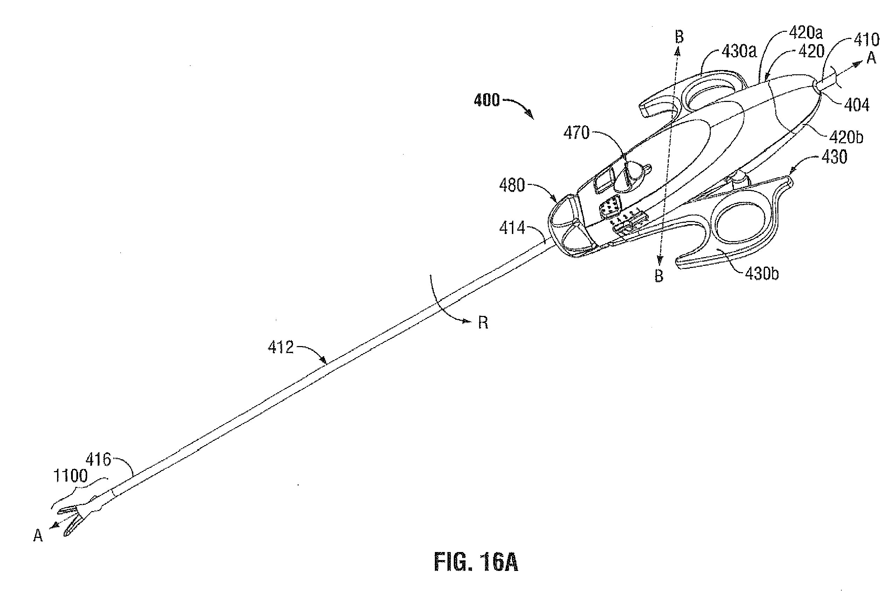

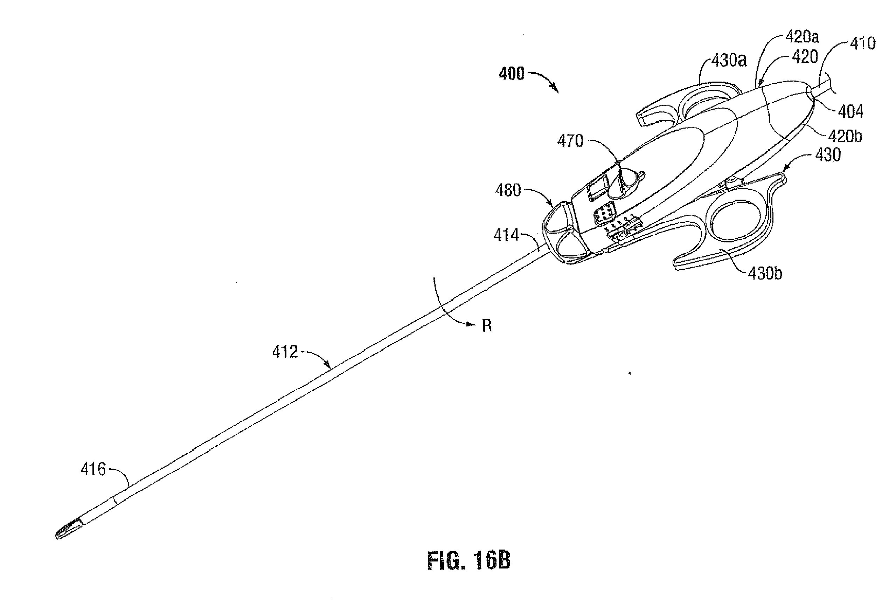

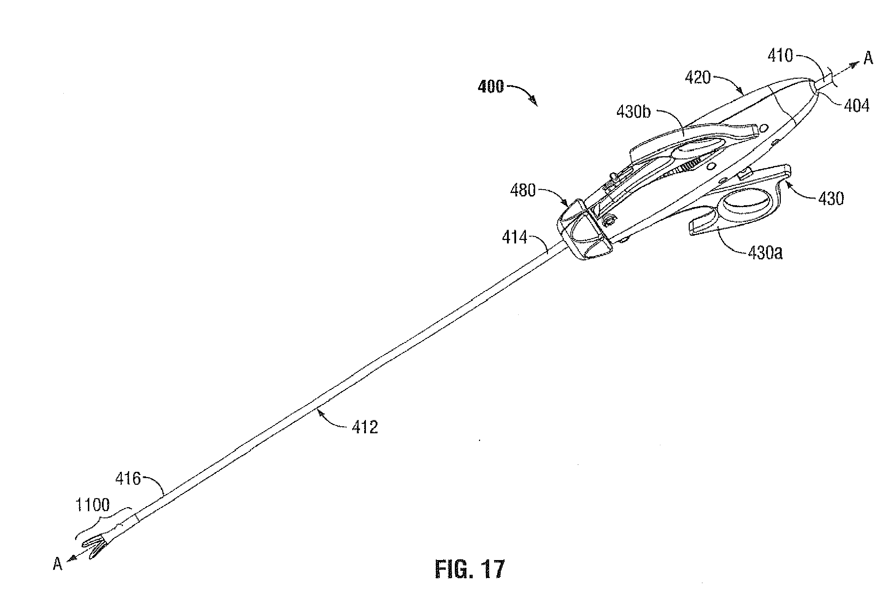

Turning now to Figs. 16A-17, one embodiment of a combination endoscopic bipolar and monopolar forceps 400 is shown for use with various surgical procedures and generally includes a housing 420, a handle assembly 430, a rotating assembly 480, a knife trigger 470 and an end effector assembly 1100 which mutually cooperate to grasp, seal and divide tubular vessels and vascular tissue (Figs. 32A and 32B).

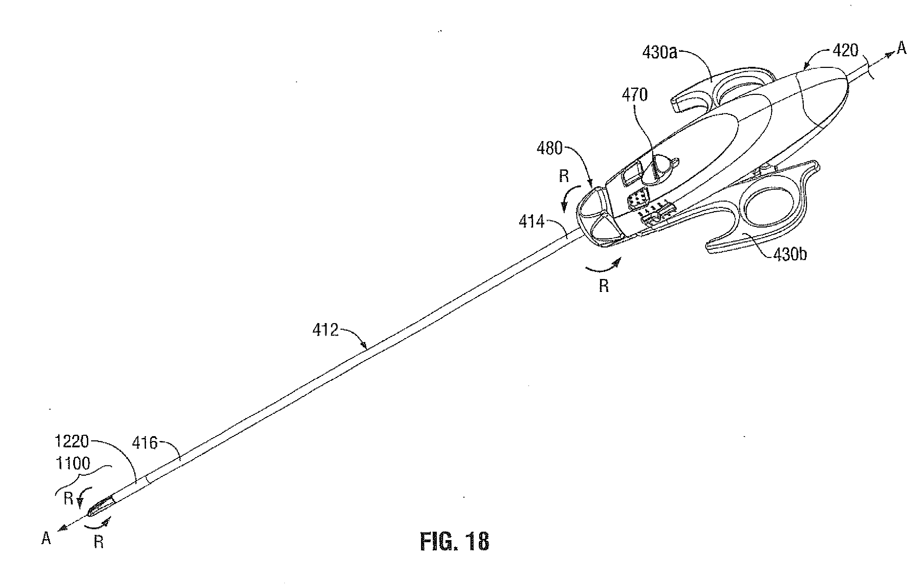

Forceps 400 includes a shaft 412 which has a distal end 416 dimensioned to mechanically engage the end effector assembly 1100 and a proximal end 414 that mechanically engages the housing 420. Handle assembly 430 includes two movable handles 430a and 430b disposed on opposite sides of housing 420. Handles 430a and 430b are movable relative to one another to actuate the end effector assembly 1100 as explained in more detail below with respect to the operation of the forceps 400. Rotating assembly 480 is mechanically coupled to housing 420 and is rotatable approximately 90 degrees in either direction about a longitudinal axis "A" (see Figs. 16A-18).

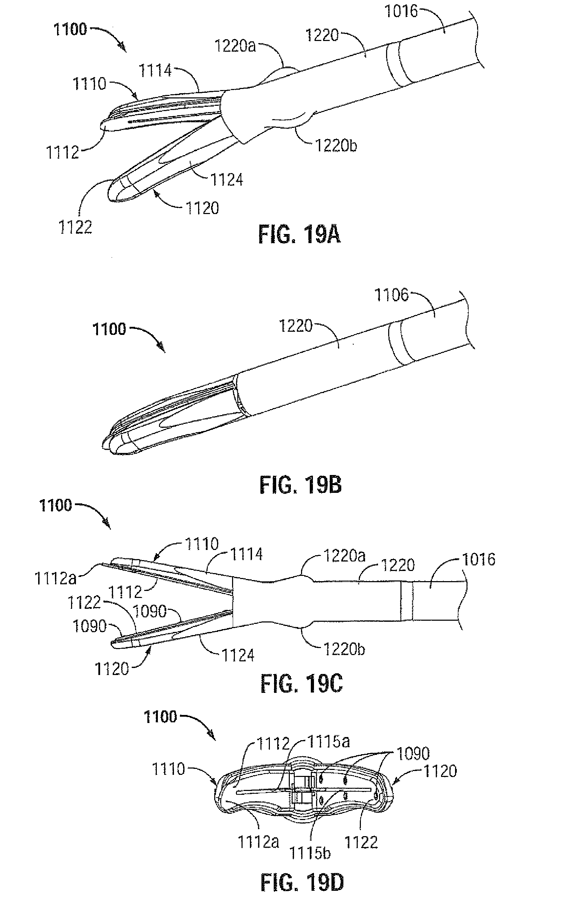

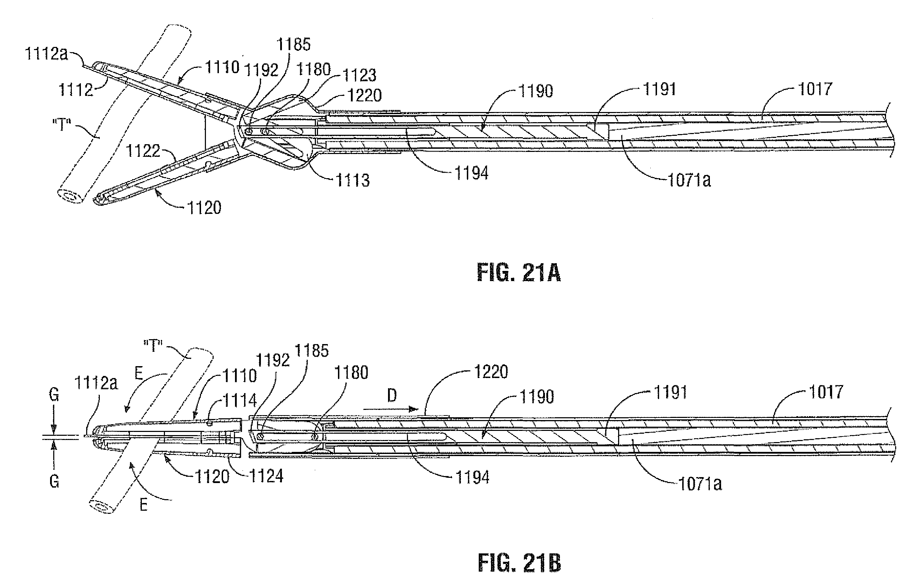

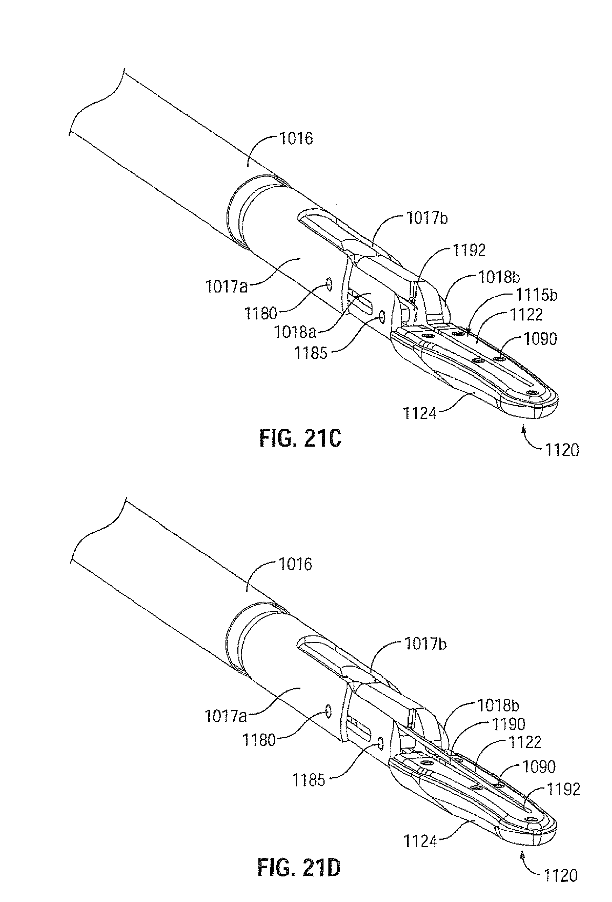

As mentioned above, end effector assembly 1100 is attached at the distal end 416 of shaft 412 and includes a pair of opposing jaw members 1110 and 1120 (See Figs. 19A-19D). Handles 430a and 430b of handle assembly 430 ultimately connect to the drive assembly in forceps 400 which, together, mechanically cooperate to impart movement of the jaw members 1110 and 1120 from an open position wherein the jaw members 1110 and 1120 are disposed in spaced relation relative to one another, to a clamping or closed position wherein the jaw members 1110 and 1120 cooperate to grasp tissue (Figs. 21A and 21B) therebetween.

Housing 420 may be similar to handpiece 36 of Fig. 3. That is, housing 420 may also include a battery, a controller and a generator. The battery supplies power to the controller and the generator and may be a primary battery or a secondary battery. The controller receives inputs from the various switches, intensity controller, nubs, potentiometers or the like that may be disposed in forceps 400 and outputs a signal to the generator. The generator provides electrosurgical energy based on the signal provided by the controller.

Similar to electrosurgical pencil 100 described above, forceps 400 includes a cable 410 coupled to return port 404. The other end of cable 410 is coupled to a return pad (not shown). Connecting the return pad directly to forceps 400 precludes the need for a separate generator and/or controller.

End effector assembly 1100 may have one or more electrodes that may be arranged in different configurations. For instance, for monopolar surgical procedures, either jaw member 1110 or 1120 may have an electrode or an electrode may be provided on knife 1190. Alternatively, two electrodes may be provided for bipolar surgical procedures where each jaw member 1110 and 1120 has an electrode. Additionally, three electrodes may be provided so that each jaw member 1110 and 1120 has an electrode and knife 1190 also has an electrode.

From the foregoing and with reference to the various figure drawings, those skilled in the art will appreciate that certain modifications can also be made to the present disclosure without departing from the scope of the same. For example, it may be preferable to add other features to the shafts described herein, e.g., an articulating assembly to axially displace the end effector assembly relative to the shaft.

It is also contemplated that the electrosurgical system (and/or the electrosurgical generator) may include a sensor or feedback mechanism (not shown) that automatically selects the appropriate amount of electrosurgical energy to effectively treat the particularly-sized tissue. The sensor or feedback mechanism may also measure the impedance across the tissue during treatment and provide an indicator (visual and/or audible) that treatment is complete.

Further, the electrosurgical devices described herein include a battery that supplies power to the various components included in the electrosurgical devices. The battery may be replaceable or rechargeable. A rechargeable battery may be removed from the device and recharged or the device may have a charging port that can be connected to a power source or placed in a receptacle to recharge the battery.

In addition, the electrosurgical energy source or generator included in the electrosurgical device may be replaceable after a single use or multiple uses. Further, the controller may also be replaceable after a single use or multiple uses.

In another embodiment of the present disclosure, battery 52, controller 48, and generator 42 may be provided as a single unit or assembly that can be easily inserted into handpiece 36 and then sealed from the environment by a door (not shown).

In another embodiment, battery 52, controller 48, and/or generator 42 may be mounted on a belt or harness worn by a user to reduce the weight of the handpiece. Battery 52, controller 48, and generator 42 may be mounted on the belt or harness as a single device or separate devices.

Additionally, battery 52, controller 48, and/or generator 42 may be incorporated into the return pad instead of the handpiece. Alternatively, battery 52, controller 48, and/or generator 42 may be strapped to a patient or to a patient support such as an operating table, gurney or stretcher.

In yet another embodiment of the present disclosure, battery 52 may be a "smart" or "intelligent" battery. The smart battery is used to power a surgical or other device, such as electrosurgical system 30. However, the smart battery is not limited to a particular type of electrosurgical device and, as will be explained, can be used in a variety of devices, which may or may not have power (i.e., current and voltage) requirements that vary from each other. The smart battery is able to identify the particular device to which it is electrically coupled. When the smart battery is inserted into handpiece 36, a connection portion makes communicating contact with a device identifier stored in memory 50. The handpiece 36, through hardware, software, or a combination thereof, is able to transmit information to the smart battery assembly. This communicated identifier is received by the connection portion of the smart battery assembly.

In one embodiment, once the smart battery assembly receives the information, the communication portion is operable to control the output of the smart battery assembly to comply with the device's specific power requirements. By integrating a microcontroller in the communication portion of the smart battery assembly, it is no longer required that a programmable device be placed in the disposable handle portion.

In one embodiment, the communication portion may include controller 48 and memory 50 (see Fig. 3), which may be separate components or a single component. The controller 48, in combination with memory 50, is able to provide intelligent power management for the electrosurgical system 30.

In another embodiment, the electrosurgical system 30 may have a plurality of buttons that can have various functions that pertain to operation of the electrosurgical system 30, e.g., activation of the device, turning the device off, selecting a device mode, selecting a display mode for display screen, or the like.

In accordance with yet another embodiment, the electrosurgical system 30 may be provided with a display screen that conveys visual information to an operator. The visual information can be, for instance, the number of uses a particular shaft has been subjected to, the battery voltage, the status of the device, such as indicating a non-engaged condition of the device components, button states, warnings, and many others.

In yet another embodiment, the display screen may be remotely positioned from electrosurgical system 30. Electrosurgical system 30 may include a wireless transmission circuit capable of wirelessly transmitting information to the remote display. Such a configuration results in a lighter handpiece used in electrosurgical system 30 while also providing a larger display screen that allows a user to see the visual information clearer. The wireless transmission circuit may also transmit information to a computer, server or any other information gathering apparatus to collect data pertaining to electrosurgical system 30. The information transmitted by electrosurgical system may be transmitted using any wireless protocol such as, but not limited to, 3G, 4G, code division multiple access (CDMA), frequency division multiple access (FDMA), Bluetooth or the like.

In another embodiment, the handpiece may be tethered to an external generator and power source. The handpiece may also have a return port that is coupled to a return pad. RF energy received by the return pad may be transmitted to the generator via the handpiece.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of preferred embodiments. Those skilled in the art will envision other modifications within the scope and spirit of the claims appended hereto.

## Claims

1. An electrosurgical device, comprising:
a housing including a cavity defined therein for housing an electrosurgical energy source, a controller configured to control the output of the electrosurgical energy source, and a power supply configured to supply power to the electrosurgical energy source and the controller, the housing including:
an active port configured to operatively couple to an end effector, wherein the end effector applies electrosurgical energy from the electrosurgical energy source to tissue; and
a return port configured to operatively couple to a return pad to provide a return path for the electrosurgical energy applied to tissue.

2. The electrosurgical device according to claim 1, wherein the electrosurgical energy source outputs the electrosurgical energy as a waveform, such as in the form of a sine waveform, a square waveform, a pulse width modulated signal or a saw tooth waveform.

3. An electrosurgical pencil, comprising:
an elongated housing including a cavity defined therein for housing an electrosurgical energy source, a controller configured to control the output of the electrosurgical energy source, and a power supply configured to supply power to the electrosurgical energy source and the controller, the elongated housing including:
a return port configured to be operatively coupled to a return pad;
an electrocautery electrode supported within the housing and extending distally from the housing, the electrocautery electrode being connected to the electrosurgical energy source; and
a plurality of activation switches supported on the housing, each activation switch being configured and adapted to selectively complete a control loop extending from the electrosurgical energy source upon actuation thereof.

4. The electrosurgical pencil according to claim 3, wherein at least one activation switch is configured and adapted to control a waveform duty cycle to achieve a desired surgical intent.

5. The electrosurgical pencil according to claim 3 or 4, further including three mode activation switches supported on the housing.

6. The electrosurgical pencil according to claim 5, wherein each mode activation switch delivers a characteristic signal to the source of electrosurgical energy which in turn transmits a corresponding waveform duty cycle to the electrosurgical pencil.

7. The electrosurgical pencil according to claim 3, 4, 5 or 6, wherein a first activation switch delivers a first characteristic signal to the source of electrosurgical energy which in turn transmits a waveform duty cycle which produces a cutting effect, a second activation switch delivers a second characteristic signal to the source of electrosurgical energy which in turn transmits a waveform duty cycle which produces a blending effect, and wherein a third activation switch delivers a third characteristic signal to the source of electrosurgical energy which in turn transmits a waveform duty cycle which produces a coagulating effect.

8. The electrosurgical device or pencil according to any one of the preceding claims, wherein the power supply is a battery.

9. The electrosurgical device or pencil according to claim 8, wherein the battery is selectively replaceable.

10. The electrosurgical device or pencil according to claim 8, wherein the battery is rechargeable.

11. An endoscopic forceps, comprising:
a housing having a shaft attached thereto, the housing including a cavity defined therein for housing an electrosurgical energy source, a controller configured to control the output of the electrosurgical energy source, a power supply configured to supply power to the electrosurgical energy source and the controller, the housing including:
a return port configured to be operatively coupled to a return pad the shaft including a pair of jaw members disposed at a distal end thereof;
a drive assembly disposed in the housing operable to move the jaw members relative to one another from a first position, wherein the jaw members are disposed in spaced relation relative to one another, to a second position, wherein the jaw members are closer to one another, for manipulating tissue;
each jaw member adapted to connect to the electrosurgical energy source such that the jaw members are capable of conducting energy for treating tissue;
a first switch disposed on the housing and being activatable to selectively deliver energy of a first electrical potential to at least one jaw member for treating tissue in a monopolar fashion; and
a second switch disposed on the housing and being activatable to selectively deliver energy of a first electrical potential to one jaw member and selectively deliver energy of a second electrical potential to the other jaw member for treating tissue in a bipolar fashion.

12. The endoscopic forceps according to claim 11 wherein the power supply is a battery.

13. The endoscopic forceps according to claim 12, wherein the battery is selectively replaceable.

14. The endoscopic forceps according to claim 12, wherein the battery is rechargeable.
